# EUROPEAN PATENT APPLICATION

(11) **EP 2 818 097 A1**
(43) Date of publication of application: **31.12.2014**
(21) Application number: 14174116.5
(22) Date of filing: 26.06.2014
(51) Int. Cl.: A61B 1/31, A61B 1/06, A61F 5/00

(54) **Improved medical device**

(30) Priority: 28.06.2013 GB 201311639
(71) Applicant: Rocket Medical PLC, Watford, Hertfordshire WD2 4XX (GB)
(72) Inventor: Jessup, Mark, Stocksfield, Northumberland NE43 7PQ (GB)
(74) Representative: Rutherford, Claire

(57) **Abstract**

A rectal probe is in the form of a one piece plastics moulding. The moulding provides a hollow body having a closed end, an open end and an opening in a wall of the hollow body situated between the open and closed ends and a handle.

## Description

The present invention relates to an improved medical device and in particular to an improved medical device adapted for use in the ligation of haemorrhoids and fluid therapy treatments.

### Background of the Invention

One type of procedure used in the treatment of haemorrhoids is known as doppler-guided haemorrhoidal artery ligation. During this procedure a probe is inserted into the rectum of the patient. The probe has an opening in a side wall thereof. The surgeon is guided by the doppler signal so that the opening may be aligned with the haemorrhoidal artery requiring ligation. The ligation procedure is performed through the opening. The doppler device is mounted in the probe. The distal end of the probe is closed, the proximal end being open. The circumferntial edge being provided with finger grip elements so that the surgeon may rotate the probe in the patient's rectum until the opening is adjacent an haemorrhoidal artery requiring ligation.
Whilst doppler-guided haemorrhoidal artery ligation is used, it has been found that surgeons can detect haemorrhoidal arteries without doppler guidance.
In addition to the performance of haemorrhoidal artery ligation there is also a need to treat rectal mucosa by fluid therapy, for example in the treatment of radiation proctitis.
It would therefore be advantageous to provide an improved medical device.

### Summary of the Invention

According to the invention there is provided a rectal probe in the form of a one piece plastics moulding, the moulding providing a hollow body having a closed end, an open end and an opening in a wall of the hollow body situated between the open and closed ends, and a handle.

Preferably, the opening is less than half the length of the hollow body.

Preferably, the hollow body includes a nose forming the closed end, a conical portion forming the open end, the nose and the conical portion joined together by a hollow shaft portion. The shaft portion is preferably cylindrical.

The opening is preferably provided in the wall of the shaft portion, and advantageously the size of the opening is less than half the length of the shaft portion, preferably between one half and one eighth of the length of the shaft portion.

Preferably, the opening is positioned closer to the closed end than to the open end.

Advantageously, the handle extends to one side of the body and preferably in the region of the open end.

Preferably, the probe includes a light source situated towards the open end of the hollow body and directing light towards the region of opening. Advantageously, the light source is positioned such that light emitted therefrom is directed forwardly of the opening.

Preferably, the light source is mounted in a light mount, which light mount is attachable to the handle of the one piece moulding.

The light mount may include a shade configured to control the direction of emission of from the light source.

Preferably, the light source is an LED and the LED is battery powered. The battery or batteries may be mounted in the handle and/or the light mount.

A part of the light source may rest on a part of the one piece moulding.

The one piece moulding is preferably transparent.

The one piece moulding is preferably fluid tight with the exception of the opening in the wall of the hollow body and the open end.

The device may be a single use device.

A method of fluid therapy comprising the steps of:
inserting the rectal probe into the rectum of a patient;
aligning the opening with a region requiring treatment;
filling the hollow body of the probe to a required level with a treatment fluid;
leaving the treatment fluid in contact with the region requiring treatment for a prescribed period of time;
removing the rectal probe from the patient.

The final step of the method of therapy may be preceded by at least partially removing the treatment fluid from the hollow body.

The method of therapy may include the step of illuminating the probe by switching on the light source.

The rectal probe of the invention is a simple, cost effective device which allows surgeons to treat haemorroidhal arteries by ligation, and also to perform fluid therapy. By providing low power LED lighting of the probe the risk of combusion is removed and the position of the probe is more easily manipulate, as the surgeon does not need to work around a power lead to the probe.

The position of the opening in the probe ensures that the area of rectal mucosa requiring treatment may be treated without the treatment fluid contacting the anus, which is not usually subjected to the effects of radiation treatment, and also which contains sensitive sensor nerve endings, which the rectal mucosa does not. In the present invention, only that part of the rectal mucosa with which the opening of the probe is aligned is subject to the fluid therapy.

### Brief Description of the Drawings

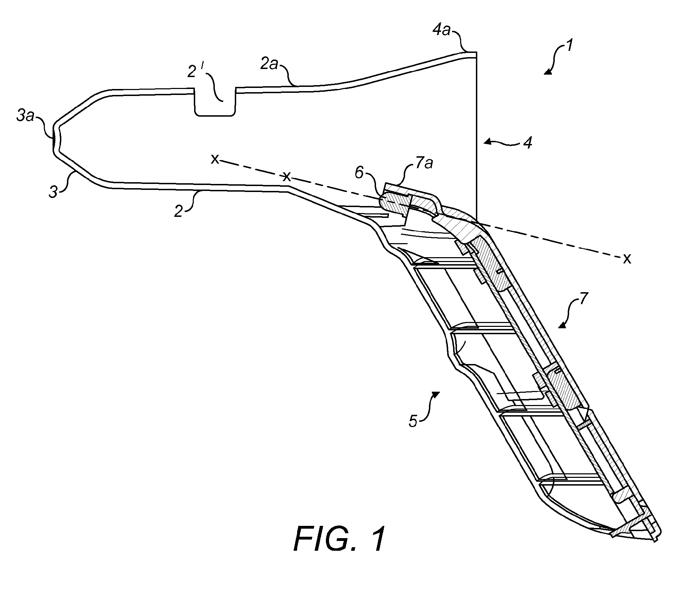

Figure 1 is a cross-sectional elevation of a preferred embodiment of a rectal probe according to the invention.

### Detailed Description of the Preferred Embodiment

Figure 1 illustrates a rectal probe 1 comprising a hollow body 2 having a closed end 3 and an open end 4 and a handle 5.

The probe 1 is formed as a one piece plastics moulding and comprises a nose portion 3a having sloping surfaces forming the closed end 3, a substantially cylindrical portion 2a and a substantially conical portion 4a, the free end of which provides the open end 4 of the probe 1.

As can be seen from Figure 1, the body 2 includes an opening 2' in the wall of the substantially cylindrical portion 2a.

The handle 5 mounts a light in the form of a low power LED 6, which is powered by one or more batteries. The LED 6 and its associated electronic circuitry are mounted in a removable element 7, which is arranged to releasably clip to the handle 5 of the one piece plastics moulding. The or each battery may be mounted in the handle 5 or the removable element 7.

As can be seen from Figure 1, the LED 6 is mounted on axis x-x. Further, the removable element 7 includes a shade portion 7a configured to shade the LED 6. The sides and base of LED 6 are therefore enclosed, ensuring that light emitted from the LED 6 is directed along axis x-x towards the region of the opneing 2'. As can be seen from Figure 1, the axis x-x intersects the wall of the probe 2 forward of the opening 2', that is in the direction of the closed end 3 of the probe 2. By so arranging the LED 6, the instruments used by the surgeon through the opening 2' are less likely to interrupt the light beam emanating from the LED 6 than would be the case if the axis x-x were aligned with the opening 2'.

Figure 1 also shows that the conical end portion 4a is asymetrical. The sloping wall of the conical end on the side of the probe from which the handle extends intersects the wall of cylindrical portion 2a forwardly of the position where the sloping wall of the conical portion intersects the wall of cylindrical portion 2a on the opposite side of the probe. The part of the conical end portion 4a forward of and adjacent the LED may include a flattened part or may be curved.

In addition or alternatively to its use in haemorroidhal artery ligation, the probe 1 may also be used in fluid treatment therapy, such as the treatment of rectal mucosa.

For example, Formalin has been shown to be effective in the treatment of radiation proctitis. This fluid is combustible. Hence, any source of ignition must be removed. Further, it is desirable to avoid any fluid leakage. The one piece nature of the probe of the present invention provides for this.

The handle allows the probe to be moved in the rectum such that window 2' is aligned with the region requiring treatment.

The LED 6 has a low power requirement and the batteries powering the LED do not represent a combustion risk.

The self-contained one-piece nature of the probe avoids fluid leakage and combustion risks, whilst providing a device which is easy for the surgeon to use.

## Claims

1. A rectal probe in the form of a one piece plastics moulding, the moulding providing a substantially hollow body having a closed end, an open end and an opening in a wall of the hollow body situated between the open and closed ends, and a handle.

2. A probe according to Claim 1, wherein the handle extends to one side of the body and preferably in the region of the open end.

3. A probe according to Claim 1 or 2, including a light source situated towards the open end of the hollow body and directing light towards the region of opening.

4. A probe according to Claim 3, wherein the light source is positioned such that light emitted therefrom is directed forwardly of the opening.

5. A probe according to Claim 3 or 4, wherein the light source is mounted in a light mount, which light mount is attachable to the handle of the one piece moulding.

6. A probe according to Claim 5, wherein the light mount includes a shade configured to control the direction of emission of from the light source.

7. A probe according to any of Claims 3 to 6, wherein a part of the light source rests on a part of the one piece moulding.

8. A probe according to any preceding claim, wherein the one piece moulding is transparent.

9. A probe according to any preceding claim, wherein the one piece moulding is fluid tight with the exception of the opening in the wall of the hollow body and the open end.

10. A probe according to any preceding claim, wherein the length of the opening is less than half the length of the hollow body.

11. A probe according to any preceding claim, the hollow body including a nose forming the closed end, a conical portion forming the open end, the nose and the conical portion joined together by a hollow shaft portion.

12. A probe according to Claim 11, wherein the opening is provided in the wall of the shaft portion.

13. A probe according to Claim 15, wherein the length of the opening is less than half the length of the shaft portion.

14. A probe according to Claim 16, wherein the length of the opening is between one half and one eighth of the lenght of the shaft portion.

15. A probe according to any preceding claim, wherein the opening is positioned closer to the closed end than to the open end.
